**Europäisches Patentamt**

**European Patent Office**

**Office européen des brevets**

(11) Veröffentlichungsnummer: **0 325 143 B1**

# EUROPÄISCHE PATENTSCHRIFT

(45) Veröffentlichungstag der Patentschrift: **23.06.93**

(51) Int. Cl.5: **C07C 31/125**, C07C 29/04

(21) Anmeldenummer: **89100333.7**

(22) Anmeldetag: **10.01.89**

(54) **Verfahren zur Herstellung von tert.-Amylalkohol (TAA).**

(30) Priorität: **19.01.88 DE 3801273**

(43) Veröffentlichungstag der Anmeldung:
**26.07.89 Patentblatt 89/30**

(45) Bekanntmachung des Hinweises auf die
Patenterteilung:
**23.06.93 Patentblatt 93/25**

(84) Benannte Vertragsstaaten:
**DE FR IT NL**

(56) Entgegenhaltungen:
**US-A- 2 477 380**
**US-A- 3 257 469**
**US-A- 4 182 920**

(73) Patentinhaber: **EC ERDÖLCHEMIE GMBH**
**Postfach 75 20 02**
**W-5000 Köln 71(DE)**

(72) Erfinder: **Schleppinghof, Bernhard, Dr.**
**Adolf-Kolping-Strasse 5**
**W-4047 Dormagen(DE)**
Erfinder: **Malessa, Reiner, Dr.**
**Naukler-Strasse 57**
**W-7400 Tübingen(DE)**
Erfinder: **Gabel, Christian, Dr.**
**Goethestrasse 69**
**W-4047 Dormagen(DE)**
Erfinder: **Scheef, Hans-Volker**
**Goethestrasse 69**
**W-4047 Dormagen(DE)**
Erfinder: **Lux, Mathias**
**Noldeweg 1**
**W-5000 Köln 30(DE)**

(74) Vertreter: **Zobel, Manfred, Dr. et al**
**c/o BAYER AG Konzernverwaltung RP Paten-**
**te Konzern**
**W-5090 Leverkusen 1 Bayerwerk (DE)**

EP 0 325 143 B1

**Beschreibung**

Die Erfindung betrifft ein Verfahren zur Herstellung von tert.-Amylalkohol (TAA) durch Hydratation von i-Amylenen an einem sauren Kationenaustauscher.

Aus US 2 813 908 ist es bereits bekannt, Olefine an Sulfonsäuregruppen enthaltenden Styrol-Divinylbenzol-Polymeren zu hydratisieren. Aus US 3 257 469 ist speziell die Herstellung von TAA an solchen sauren, Sulfonsäuregruppen enthaltenden Ionenaustauschern durch Umsetzung von i-Amylen mit einem molaren Überschuß an Wasser bekannt. Hierzu wird beispielsweise bei einem Druck von 35 bar und Temperaturen von 66 - 107°C gearbeitet. Ein für industrielle Zwecke ausreichender Anteil von TAA im Reaktionsprodukt wird durch die Mitverwendung von Lösungsmitteln erreicht, von denen i-Propanol bzw. Aceton besonders herausgestellt werden. Dieses Verfahren umfaßt damit notwendigerweise die Abtrennung des mitverwendeten Lösungsmittels aus dem Reaktionsgemisch.

US 4 182 920 stellt eine Fortentwicklung des zuletzt beschriebenen Verfahrens dar und richtet sich ebenfalls auf die Herstellung von TAA. Als neu wird herausgestellt, daß das gesamte Einsatzgemisch nur eine homogene Phase bildet und ein Teil des i-Amylens in einen zweiten von mindestens insgesamt zwei Reaktoren eingespeist wird. Als Lösungsmittel wird speziell Aceton herausgestellt, das ausweislich der Ausführungsbeispiele in einer Menge von 60-75 Gew.-% des gesamten Einsatzstroms vorliegt. Dieses Verfahren wird ebenfalls mit überschüssigen molaren Mengen an Hydratationswasser durchgeführt.

Es ist weiterhin allgemein bekannt, daß die Hydratation von i-Buten zu hohen Mengen von tert.-Butanol im Reaktionsgemisch führt, daß aber beim Übergang auf i-Amylene wegen der ungünstigen Gleichgewichtslage wesentlich geringere Mengen an TAA im Reaktionsgemisch entstehen.

Es wurde nun überraschend gefunden, daß wesentlich höhere Anteile an TAA im Reaktionsgemisch erhalten werden, wenn die Hydratation in einem Bereich von 5° unterhalb bis 5° oberhalb des Siedepunktes der i-Amylene durchgeführt wird. Das erfindungsgemäße Verfahren kommt ohne den Zusatz reaktionsfremder Stoffe, insbesondere ohne den Zusatz von Lösungsmitteln, aus.

Die Erfindung betrifft daher ein Verfahren zur Herstellung von tert.-Amylalkohol (TAA) durch Hydratation von i-Amylenen an einem sauren Kationenaustauscher, das dadurch gekennzeichnet ist, daß man in einem Bereich von 5° unterhalb bis 5° oberhalb des Siedepunktes der i-Amylene unter dem eingestellten Reaktionsdruck arbeitet.

Das erfindungsgemäße Verfahren ist dadurch gekennzeichnet, daß es in einem Bereich von 5° unterhalb bis 5° oberhalb des Siedepunktes der i-Amylene bei dem jeweils eingestellten Reaktionsdruck durchgeführt wird. In bevorzugter Weise wird in einem Bereich von 5° unterhalb bis 2° oberhalb des Siedepunktes der i-Amylene, in besonders bevorzugter Weise in einem Bereich von 2° unterhalb bis 2° oberhalb des Siedepunktes, gearbeitet. Die Einstellung des Siedepunktes der i-Amylene in Abhängigkeit vom Druck ist dem Fachmann bekannt. Erfindungsgemäß kann hierdurch der Siedepunkt der i-Amylene in den Bereich von 20-80°C, bevorzugt 35-50°C, gelegt werden. Eine ganz besonders bevorzugte Verfahrensvariante ist das Arbeiten unter Normaldruck wegen der damit verbundenen Vereinfachung der benötigten Apparate.

Als saure Kationenaustauscher können alle bekannten Typen, wie sulfonierte Phenol-Formaldehyd-Harze, sulfonierte Cumaron-Inden-Kondensationsprodukte, sulfonierte Polystyrole, sulfonierte Styrol-Divinylbenzol-Harze usw. eingesetzt werden; sie werden erfindungsgemäß in ihrer $H^+$-Form eingesetzt. In bevorzugter Weise werden erfindungsgemäß sulfonierte Styrol-Divinylbenzol-Harze mit einem Vernetzungsgrad (Gehalt an Divinylbenzol) von 2 bis 65 %, bevorzugt 8-25 %, eingesetzt. Solche sauren Kationenaustauscher sind dem Fachmann bekannt und unter vielen Bezeichnungen im Handel.

Die sauren Kationenaustauscher als Hydratationskatalysatoren werden mit einer Menge an gesamtem Einsatzgemisch beaufschlagt, die eine Raumgeschwindigkeit (LHSV = liquid hourly space velocity) von 0,05-1 l Reaktionsgemisch pro Liter Katalysator und Stunde, bevorzugt 0,1 bis 0,4 l/l • h bedeutet.

Die Wassermenge im erfindungsgemäßen Verfahren ist grundsätzlich unkritisch, so daß auch ein molarer Überschuß an Wasser tolerierbar ist. Die Untergrenze der Wassermenge wird im allgemeinen so angesetzt, daß im wesentlichen alle i-Amylene zum TAA umgesetzt werden, soweit dies die Lage des Umsetzungsgleichgewichtes erlaubt. Wird diese Mindestmenge unterschritten, kommt es zu unnötigen Minderausbeuten, die umso schmerzlicher sind, als die Gleichgewichtslage ohnehin keinen vollständigen Umsatz der i-Amylene erlaubt. In bevorzugter Weise werden daher 80-120 %, besonders bevorzugt 90-115 %, ganz besonders bevorzugt 95 bis 105 % der Wassermenge eingesetzt, die zu einer nach der Lage des Gleichgewichts theoretisch möglichen Hydratation führt. Die Lage des Gleichgewichts ist von der gewählten Reaktionstemperatur abhängig, so daß die genannten prozentualen Wassermengen dieser Gleichgewichtslage angepaßt werden müssen. Die Veränderlichkeit des Gleichgewichts mit der Reaktionstemperatur ist dem Fachmann bekannt, so daß er durch orientierende Versuche den zu wählenden prozentualen Bereich der

2

Wassermenge bestimmen kann. Zur Erhöhung der Raum-Zeit-Ausbeute kann es auch günstig sein, Verweilzeiten einzustellen, die nicht ganz zur Einstellung des Gleichgewichts ausreichen. Dann wird die benötigte Wassermenge auf den erzielbaren Umsatz an i-Amylenen berechnet. Für den Fall, daß statt in einem Reaktor in zwei oder mehreren gearbeitet wird, kann es vorteilhaft sein, das gesamte Reaktionswasser (80-120 %, bezogen auf den theoretisch zu erwartenden Gleichgewichtszustand) nur teilweise, beispielsweise zu 60 bis 90 Relativprozent des obigen prozentualen Bereiches, dem ersten Reaktor zuzusetzen, während der Rest dem zweiten oder dritten Reaktor zugesetzt wird.

Die zu hydratisierenden i-Amylene (2-Methyl-buten(1) und 2-Methyl-buten(2)) können beispielsweise als $C_5$-Destillationsschnitt (sogenannter Benzol-Vorlauf) oder in besonders bevorzugter Weise als angereicherte oder sogar im wesentlichen reine i-Amylene, beispielsweise aus der Spaltung von tert.-Amyl-methyl-ether, eingesetzt werden.

Das erfindungsgemäße Verfahren läßt höhere Gehalte an TAA im Reaktionsgemisch zu, als Verfahren des Standes der Technik ermöglichen. Solche höheren Gehalte liegen bei 25-60 Massen-%, typischerweise bei 40-50 Massen-%, bezogen auf die Summe aus TAA und i-Amylene (im Fall des Einsatzes reiner i-Amylene bezogen auf das gesamte Reaktionsprodukt). Überraschenderweise werden im erfindungsgemäßen Verfahren wesentlich geringere Gehalte an $C_5$-Dimeren beobachtet als nach Verfahren des Standes der Technik. Typische Gehalte an solchen Dimeren sind 0,01-2 %, verglichen mit 5-9 Massen-% in Verfahren des Standes der Technik (US 4 447 668).

Es wurde jedoch gefunden, daß der Gehalt an Dimeren noch weiter gesenkt werden kann, wenn man dem Einsatzgemisch 0,2-5 Gew.-%, bevorzugt 0,3-4 Gew.-%, besonders bevorzugt 1-3 Gew.-% TAA zusetzt, wobei alle Mengen auf das Gewicht des zu hydratisierenden i-Amylens bezogen sind. Es ist sehr überraschend, daß die grundsätzliche Gegenwart des TAA im Reaktionsgemisch nicht ausreicht, um die Bildung der Oligomeren und damit die Notwendigkeit ihrer Abtrennung noch weiter zu verhindern, sondern daß es notwendig ist, TAA in der beschriebenen kleinen Menge bereits vor der Zuführung zum Hydratationskatalysator im Reaktionsgemisch zu haben. Der zuzusetzende TAA kann grundsätzlich in reiner Form zugesetzt werden. Da jedoch das Umsetzungsgemisch nach Verlassen des Reaktors diesen zuzusetzenden TAA als das gewünschte Reaktionsprodukt enthält, ist es eine vorteilhafte Variante, dem Einsatzgemisch eine solche Menge des im Verfahren anfallenden Reaktionsgemisches zuzusetzen, daß die gewünschte Menge an TAA vor Beginn der katalytischen Hydratation vorliegt.

Die Ausführungsbeispiele zeigen Ausführungsvarianten, ohne die Erfindung darauf zu beschränken.

Beispiel 1 (vgl. Tabelle)

tert.-Amylalkohol (TAA) wurde durch Umsetzung von i-Amylenen und $H_2O$ bei 37 °C und Normaldruck unter Verwendung eines saure Sulfonsäuregruppen enthaltenden StyrolDivinylbenzol-Harzes (Handelsprodukt SPC 118 der Bayer AG) im kontinuierlich betriebenen Rohrreaktor hergestellt. Die Apparatur bestand aus einer mantelbeheizte Kolonne, in der der Kationenaustauscher fest angeordnet war. Am Kopf der Kolonne befand sich ein Kühler, der ein Entweichen der i-Amylene verhinderte. Am Kopf der Kolonne befanden sich weiterhin Einspeisungsstellen für Wasser und die i-Amylene. Das eingespeiste Gemisch rieselte über den Kationenaustauscher und verdampfte teilweise. Am Sumpf der Kolonne befand sich ein Sammelgefäß für umgesetztes Reaktionsgemisch. Bei einer LHSV von 0,4 wurde ein Reaktionsgemisch mit 24 % TAA erhalten.

Beispiel 2 (vgl. Tabelle)

Beispiel 1 wurde mit den Unterschied wiederholt, daß die LHSV von 0,4 auf 0,1 gesenkt wurde (vgl. Tabelle). Der TAA-Gehalt im Reaktionsgemisch wurde dadurch auf 51 % erhöht.

Tabelle

| TAA-Synthese unter Normaldruck | | |
|---|---|---|
| | Beispiel 1 | Beispiel 2 |
| LHSV ($h^{-1}$) | 0,4 | 0,1 |
| Temp. (°C) | 37°C | 37°C |
| Molverhältnis von $H_2O$/i-Amylenen im Einsatz | 1:4,9 | 1:2,2 |
| TAA im Produkt (Massen-%) | 24 % | 51 % |

Beispiele 3-5

Die Apparatur bestand aus einem Glasrohr mit eingebauter Hülsenhalterung für eine Soxhlethülse zur Aufnahme des Kationenaustauschers, einem Kolonnenkopf, der sich auf totalen Rücklauf einstellen ließ, und einen Umlaufverdampfer mit Zwangsumlauf am Kolonnensumpf; der Zwangsumlauf erfaßte die Wasserphase als die schwerste und am tiefsten gelegene im System. Oberhalb der wäßrigen Phase befand sich eine organische Phase aus der TAA entnommen wurde. Zwischen Soxhlethülse und Kolonnenkopf wurden i-Amylene eingespeist, die gemeinsam mit Kondensat aus dem Kollonenkopf über den Kationenaustauscher in der Hülse rieselten. Vom Sumpf wurden i-Amylene, Wasser und TAA als Azeotrop verdampft. Es wurden i-Amylene mit einer Reinheit von > 99 % (gaschromatographisch) eingesetzt.

Beispiel 3

In die Soxhlethülse wurden 100 ml Kationenaustauscher SPC 118 wie im Beispiel 1 eingefüllt.

Im Sumpf wurden:

140,0 g i-Amylen
72,0 g Wasser (Molverhältnis 1.0:2,0) vorgelegt.
Es bildete sich eine wäßrige und eine organische Phase.
Der Sumpfumlauf wurde angestellt und aufgeheizt. Das gesamte Destillat wurde über den unendlichen Rücklauf durch das Katalysatorbett in den Sumpf zurückgeführt.
Nach drei Stunden wurde die Apparatur kontinuierlich betrieben.

Einspeisung:

30,0 ml/h i-Amylen in den Rücklauf
5,0 ml/h Wasser in die Wasserphase des Sumpfes
Molverhältnis Wasser / i-Amylen 0,97

Abzug aus dem Sumpf:

32,0 ml/h Reaktionsprodukt aus der organischen Phase
3,0 ml/h Wasser aus der wäßrigen Phase

| Sumpftemperatur | 40,0 °C |
|---|---|
| Kopftemperatur | 37,0 °C |
| Kolonnendruck | 1,0 bar |
| Rückflußverhältnis (R/E) | unendlich |
| LHSV (liquid hourly space velocity) | 0,35 |
| Versuchsdauer | 236,0 Stunden |

Produktverteilung (Beispiel 3):

| | Stelle | | |
|---|---|---|---|
| Einspeisung | Kopf | Sumpf | Summe |
| Komponente | | | |
| i-Amylen (g/h) | 20,0 | | 20,0 |
| Wasser (g/h) | 5,0 | | 5,0 |
| Gesamt (g/h) | 25,0 | | 25,0 |
| Entnahme | | | |
| Komponente | | | |
| i-Amylen (g/h) | | 10,4 | 10,4 |
| TAA (g/h) | | 11,6 | 11,6 |
| Höhere (g/h) | | 0,05 | 0,05 |
| Rest-KW (g/h) | | 0,3 | 0,3 |
| Wasser (g/h) | | 2,65 | 2,65 |
| Gesamt (g/h) | | | 25,00 |

Ausbeute TAA : = 11,6 g (0,13 Mol)
= 46,1 %

Umsatz i-Amylen : 20,0 - 10,4 = 9,6 g
= 48,0 %

Beispiel 4

Der Versuch wurde analog zu Beispiel 3 durchgeführt.
Der Wassereinsatz wurde entsprechend dem Umsatz minimiert, es wurde kein Wasser abgezogen.
Die laufende Apparatur wurde auf folgende Werte umgestellt:

Einspeisung:

30,0 ml/h i-Amylen in den Rücklauf
2,0 ml/h Wasser in die Wasserphase des Sumpfes

Abzug aus dem Sumpf:

32,0 ml/h Reaktionsprodukt aus der organischen Phase

| | |
|---|---|
| Sumpftemperatur | 40,0 °C |
| Kopftemperatur | 37,0 °C |
| Kolonnendruck | 1,0 bar |
| Rückflußverhältnis (R/E) | unendlich |
| LHSV (liquid hourly space velocity) | 0,32 |
| Versuchsdauer | 90,0 Stunden |

Produktverteilung (Beispiel 4):

| | | Stelle | | |
|---|---|---|---|---|
| | Komponente | Kopf | Sumpf | Summe |
| Einspeisung | i-Amylen (g/h) | 20,0 | | 20,0 |
| | Wasser (g/h) | | 2,0 | 2,0 |
| | Gesamt (g/h) | | | 22,0 |
| Entnahme | i-Amylen (g/h) | | 10,2 | 10,2 |
| | TAA (g/h) | | 11,5 | 11,5 |
| | Höhere (g/h) | | 0,03 | 0,03 |
| | Rest-KW (g/h) | | 0,27 | 0,27 |
| | Gesamt (g/h) | | | 22,00 |

Ausbeute TAA : = 11,5 g (0,13 Mol) = 46,0 %

Umsatz i-Amylen : 20,0 - 10,2 = 9,8 g = 49,0 %

7

Beispiel 5

Der Versuch wurde analog zu Beispiel 3 durchgeführt.
Es wurde der i-Amylen- und der Wasserdurchsatz erhöht um eine höhere LHSV zu erreichen.
Die laufende Apparatur wurde auf folgende Werte umgestellt:

Einspeisung:

50,0 ml/h i-Amylen in den Rücklauf
8,0 ml/h Wasser in die Wasserphase des Sumpfes

Abzug aus dem Sumpf:

53,0 ml/h Reaktionsprodukt aus der organischen Phase
5,5 ml/h Wasser aus der wäßrigen Phase

## Produktverteilung (Versuch 5):

| | | | Stelle | | |
|---|---|---|---|---|---|
| | Einspeisung | | Kopf | Sumpf | Summe |
| Komponente | | | | | |
| i-Amylen (g/h) | 33,0 | | 33,0 | | 33,0 |
| Wasser (g/h) | 8,0 | | | 8,0 | 8,0 |
| Gesamt (g/h) | 41,0 | | | | 41,0 |
| | Entnahme | | | | |
| Komponente | | | | | |
| i-Amylen (g/h) | 22,8 | | | 22,8 | 22,8 |
| TAA (g/h) | 12,5 | | | 12,5 | 12,5 |
| Höhere (g/h) | < 0,01 | | | < 0,01 | < 0,01 |
| Rest-KW (g/h) | 0,2 | | | 0,2 | 0,2 |
| Wasser (g/h) | 5,5 | | | 5,5 | 5,5 |
| Gesamt (g/h) | 41,0 | | | | 41,00 |

Ausbeute TAA      :                              =      12,5 g (0,14 Mol)
                                                 =      30,0 %

Umsatz i-Amylen :      33,0      -      22,8      =      10,2 g
                                                 =      30,8 %

| Sumpftemperatur | 40 °C |
|---|---|
| Kopftemperatur | 37 °C |
| Kolonnendruck | 1,0 bar |
| Rückflußverhältnis (R/E) | unendlich |
| LHSV (liquid hourly space velocity) | 0,60 |
| Versuchsdauer | 220,0 Stunden |

**Patentansprüche**

1. Verfahren zur Herstellung von tert.-Amylalkohol (TAA) durch Hydratation von i-Amylenen an einem sauren Kationenaustauscher, dadurch gekennzeichnet, daß man in einem Bereich von 5° unterhalb bis 5° oberhalb des Siedepunktes der i-Amylene unter dem eingestellten Reaktionsdruck arbeitet.

2. Verfahren nach Anspruch 1, dadurch gekennzeichnet, daß man in einem Bereich von 5° unterhalb bis 2° oberhalb des Siedepunktes der i-Amylene arbeitet.

3. Verfahren nach Anspruch 2, dadurch gekennzeichnet, daß man in einem Bereich von 2° unterhalb bis 2° oberhalb des Siedepunktes der i-Amylene arbeitet.

4. Verfahren nach Anspruch 1, dadurch gekennzeichnet, daß man den Siedepunkt der i-Amylene durch an sich bekannte Druckeinstellung in den Bereich von 20 bis 80°C, bevorzugt 35 bis 50°C, legt.

5. Verfahren nach Anspruch 4, dadurch gekennzeichnet, daß man in einem Bereich von 5° unterhalb bis 5° oberhalb des Siedepunktes der i-Amylene unter Normaldruck arbeitet.

6. Verfahren nach Anspruch 1, dadurch gekennzeichnet, daß eine Raumgeschwindigkeit (LHSV = liquid hourly space velocity) von 0,05 bis 1 Liter Einsatzgemisch pro Liter Katalysator und Stunde eingestellt wird.

7. Verfahren nach Anspruch 1, dadurch gekennzeichnet, daß das Hydratationswasser in einer molaren Menge von 80 bis 120 %, bevorzugt 90 bis 115 %, besonders bevorzugt 95 bis 105 %, bezogen auf die zu erwartende Menge an TAA, eingesetzt wird.

8. Verfahren nach Anspruch 7, dadurch gekennzeichnet, daß bei mehrstufiger Verfahrensdurchführung 60 bis 90 % des gesamten Reaktionswassers vor der ersten Verfahrensstufe und der Rest nach der ersten Verfahrensstufe zugesetzt werden.

9. Verfahren nach Anspruch 1, dadurch gekennzeichnet, daß dem Einsatzgemisch 0,2 bis 5 Gew.-%, bezogen auf das Gewicht der i-Amylene, an TAA zugesetzt werden.

**Claims**

1. Process for the preparation of tert.-amyl alcohol (TAA) by hydration of i-amylenes on an acid cation exchanger, characterised in that the reaction is carried out in a range from 5° below to 5° above the boiling point of the i-amylenes under the set reaction pressure.

2. Process according to Claim 1, characterised in that the reaction is carried out in a range from 5° below to 2° above the boiling point of the i-amylenes.

3. Process according to Claim 2, characterised in that the reaction is carried out in a range from 2° below to 2° above the boiling point of the i-amylenes.

4. Process according to Claim 1, characterised in that the boiling point of the i-amylenes is set in the range from 20 to 80°C, preferably 35 to 50°C, by adjusting the pressure in a manner known per se.

5. Process according to Claim 4, characterised in that the reaction is carried out in a range from 5° below to 5° above the boiling point of the i-amylenes under atmospheric pressure.

6. Process according to Claim 1, characterised in that a space velocity (LHSV = liquid hourly space velocity) from 0.05 to 1 litre of the starting mixture per litre of catalyst and hour is set.

7. Process according to Claim 1, characterised in that the water of hydration is used in a molar amount from 80 to 120%, preferably 90 to 115%, particularly preferably 95 to 105%, relative to the amount of TAA expected.

**8.** Process according to Claim 7, characterised in that, if the process is carried out in several steps, 60 to 90% of the total reaction water is added before the first process step and the remainder after the first process step.

**9.** Process according to Claim 1, characterised in that 0.2 to 5% by weight, relative to the weight of the i-amylenes, of TAA is added to the starting mixture.

**Revendications**

**1.** Procédé de production d'acide tertio-amylique (TAA) par hydratation d'iso-amylènes sur un échangeur cationique acide, caractérisé en ce qu'on opère dans une plage allant de 5° au-dessous jusqu'à 5° au-dessus du point d'ébullition des iso-amylènes à la pression réactionnelle réglée.

**2.** Procédé suivant la revendication 1, caractérisé en ce qu'on opère dans une plage allant de 5° au-dessous à 2° au-dessus du point d'ébullition des iso-amylènes.

**3.** Procédé suivant la revendication 2, caractérisé en ce qu'on opère dans une plage allant de 2° au-dessous à 2° au-dessus du point d'ébullition des iso-amylènes.

**4.** Procédé suivant la revendication 1, caractérisé en ce qu'on ajuste le point d'ébullition des iso-amylènes par un réglage de pression d'une manière connue dans la plage de 20 à 80°C, de préférence de 35 à 50°C.

**5.** Procédé suivant la revendication 4, caractérisé en ce qu'on opère dans une plage allant de 5° au-dessous à 5° au-dessus du point d'ébullition des iso-amylènes, à la pression normale.

**6.** Procédé suivant la revendication 1, caractérisé en ce qu'on règle une vitesse spatiale (VSHL) = liquide hourly space velocity) de 0,05 à 1 litre de mélange utilisé par litre de catalyseur et par heure.

**7.** Procédé suivant la revendication 1, caractérisé en ce qu'on utilise l'eau d'hydratation en une quantité molaire de 80 à 120 %, de préférence de 90 à 115 %, notamment de 95 à 105 % par rapport à la quantité de TAA à laquelle on doit s'attendre.

**8.** Procédé suivant la revendication 7, caractérisé en ce que lors de sa mise en oeuvre en plusieurs étapes, on ajoute 60 à 90 % de l'eau totale de réaction avant la première étape du procédé et le reste après cette première étape.

**9.** Procédé suivant la revendication 1, caractérisé en ce qu'on ajoute au mélange chargé 0,2 à 5 % en poids de TAA par rapport au poids des iso-amylènes.